# EUROPEAN PATENT SPECIFICATION

(11) **EP 3 700 463 B1**
(45) Date of publication and mention of the grant of the patent: **21.06.2023**
(21) Application number: 18792943.5
(22) Date of filing: 26.10.2018
(51) Int. Cl.: A61F 2/01

(54) **A DOME SHAPED FILTERING DEVICE**
KUPPELFÖRMIGE FILTERVORRICHTUNG
DISPOSITIF DE FILTRATION EN FORME DE DÔME

(30) Priority: 27.10.2017 EP 17199058
(43) Date of publication of application: 02.09.2020
(73) Proprietor: Keystone Heart Ltd., 3088900 Caesarea (IL)
(72) Inventor: ASHKENAZI, Amit, 3465316 Haifa (IL); PONOMARENKO, Valentin, 34367 Haifa (IL)
(74) Representative: KIPA AB
(86) International application number: PCT/EP2018/079360
(87) International publication number: WO 2019/081689

(56) References cited:
- EP-A1- 2 859 864
- WO-A1-96/01591
- US-A1- 2004 153 119

## Description

### BACKGROUND OF THE INVENTION

### Field of the Invention

This disclosure pertains in general to a dome-shaped intra-aortic filtering devices and methods to prevent emboli from entering arteries branching from the aorta, e.g., arteries that lead to the brain. In particular, the disclosure relates to manufacturing of a dome-shaped intra-aortic filtering devices.

### Background of the Disclosure

Particles such as emboli may form, for example, as a result of the presence of particulate matter in the bloodstream. Particulate matter may originate from for example a blood clot occurring in the heart. The particulate may be a foreign body, but may also be derived from body tissues. For example, atherosclerosis, or hardening of the blood vessels from fatty and calcified deposits, may cause particulate emboli to form. Moreover, clots can form on the luminal surface of the atheroma, as platelets, fibrin, red blood cells and activated clotting factors may adhere to the surface of blood vessels to form a clot.

Blood clots or thrombi may also form in the veins of subjects who are immobilized, particularly in the legs of bedridden or other immobilized patients. These clots may then travel in the bloodstream, potentially to the arteries of the lungs, leading to a common, often-deadly disease called pulmonary embolus. Thrombus formation, and subsequent movement to form an embolus, may occur in the heart or other parts of the arterial system, causing acute reduction of blood supply and hence ischemia. The ischemia damage often leads to tissue necrosis of organs such as the kidneys, retina, bowel, heart, limbs, brain or other organs, or even death.

Since emboli are typically particulate in nature, various types of filters have been proposed in an attempt to remove or divert such particles from the bloodstream before they can cause damage to bodily tissues.

Various medical procedures may perturb blood vessels or surrounding tissues. When this occurs, potentially harmful particulates, such as emboli, may be released into the blood stream. These particulates can be damaging, e.g., if they restrict blood flow to the brain. Devices to block or divert particulates from flowing into particular regions of the vasculature have been proposed but may not eliminate the risks associated with the release of potentially harmful particulates into the blood stream during or after particular medical procedures.

Improved devices for blocking or diverting vascular particulates are under development, but each intravascular procedure presents unique risks.

As intravascular devices and procedures, such as transcatheter aortic valve implantation (TAVI), become more advanced, there is an emerging need for features that provide these devices with improved ease of use, intravascular stability, and embolic protection.

WO 96/01591 describes intravascular devices for treating certain medical conditions and provides a method of forming intravascular devices and certain intravascular occlusion devices.

EP 2 859 864 A1 describes a medical device for embolic protection in an aortic arch, comprising a catheter having a shaft and a distal end portion of the shaft, an expandable embolic protection device having a filter membrane and a frame, wherein the frame comprises a frame loop and an elongated frame shaft having a distal end portion connected to the frame loop in a connection point.

US 2004/0153119 A1 describes a device for filtering emboli from blood flowing through a lumen defined by the walls of a vessel in a patient's body, comprising a filter element and a self-expanding radial element associated with the filter element. The filter element is expandable from a collapsed configuration when the filter element is restrained to an expanded configuration when the filter element is unrestrained.

Possible areas of improvements of such devices and procedures include "windsailing" of devices with pulsatile blood flow, leakage of fluid and/or particulate matter at peripheral portions of devices during use thereof, secure positioning in a patient during use and/or retrievability, etc.

Hence, an improved intravascular device, system and/or method would be advantageous and in particular allowing for increased flexibility, cost-effectiveness, and/or patient safety would be advantageous.

### SUMMARY OF THE DISCLOSURE

Accordingly, examples of the present disclosure preferably seek to mitigate, alleviate or eliminate one or more deficiencies, disadvantages or issues in the art, such as the above-identified, singly or in any combination by providing a device according to the appended patent claims.

The invention relates to an embolic protection device for deployment in an aortic arch of a patient for protection of side branch vessels of the aortic arch from embolic material according to independent claim 1. The device comprises a filter membrane having a 3D-structure molded using heat treatment.

The device also includes a support frame along the periphery of said 3D-structure.

The support frame stretchs the filter membrane to become substantially flat when unconstrained and the support frame is fully expanded.

The filter membrane obtains a pre-set 3D-structure when the support frame is constrained, such as when deployed in the aortic arch and the support frame is constrained by the interior walls of the aortic arch.

Further examples of the embolic protection device are disclosed in accordance with the description and the dependent claims.

It should be emphasized that the term "comprises/comprising" when used in this specification is taken to specify the presence of stated features, integers, steps or components but does not preclude the presence or addition of one or more other features, integers, steps, components or groups thereof.

### BRIEF DESCRIPTION OF THE DRAWINGS

These and other aspects, features and advantages of which examples of the disclosure are capable of will be apparent and elucidated from the following description of examples of the present disclosure, reference being made to the accompanying drawings, in which the schematic illustrations of
Figs. 1A to 1B are illustrating schematic examples of a 3-dimensional filter unit manufactured using the disclosed heat treatment method and before being mounted on a support frame;
Figs. 2A and 2B are illustrating one exemplary part of a mold having a cavity in the shape of the 3-Dimensional (3D) structure;
Figs. 2C and 2D are illustrating one exemplary part of a mold having a protrusion in the shape of the 3-Dimensional (3D) structure;
Figs. 2E and 2F are illustrating the exemplary mold when the two exemplary parts of Figs. 2A to 2D are closed;
Fig. 3 is illustrating an example of determining the amount of fabric needed in the mold before heat-treatment.
Fig. 4 is illustrating exemplary characteristic of a heat-treated 3D-strucuture;
Figs. 5A to 5D are illustrating a schematic example of an embolic protection device in accordance with the invention having a filter membrane with a 3D-structure when unconstrained and constrained;
Fig. 6 is illustrating a schematic example of an embolic protection device in figs. 5A to 5D when deployed in an aortic arch;
Figs. 7A and 7B are illustrating an example of a jig for holding and constraining a support member when a heat-treated filter is mounted;
Fig. 8 is illustrating a chart over the manufacturing method described herein.

### DESCRIPTION OF EXAMPLES

The following disclosure focuses on examples of the present disclosure applicable to a method of manufacturing an emboli filter having a three-dimensional (3D) structure from a flat 2-dimensional sheet of fabric made from at least of strand. The disclosure further relates to an embolic protection device, such as a collapsible embolic protection device, for delivery to an aortic arch of a patient for protection of side branch vessels of the aortic arch from embolic material. In particular, an embolic protection device having a filter membrane with a three-dimensional structure manufactured according to the disclosed method. The filter is preferably delivered through transvasculary. In some alternative examples, the device may be configured to be delivered through a side branch vessels of the aortic arch.

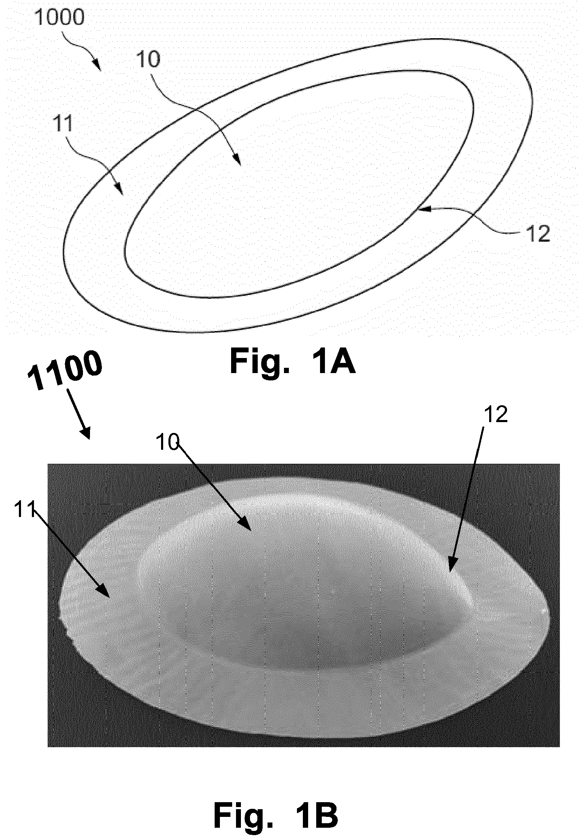

Fig. 1A is a schematic illustration of a filter membrane 1000 having a 3-dimensional structure obtained by the disclosed manufacturing method and before being mounted on a support member. Fig. 1B is an exemplary picture of a filter membrane 1100 manufactured using the disclosed manufacturing method and before being mounted on a support member. The 3-dimensional structure may be made to cover the whole filter area of the embolic protection device. Alternatively, the 3-dimensional structure may be made to cover only a part of the embolic protection device, such as a proximal portion, a distal portion, or a central portion.

The 3-dimensional structure has a concave inner surface and may have a convex-like outer curvature. The form of the 3-dimensional structure improves the adaptation of the filter membrane to the anatomy of the aortic arch and to provide a better apposition to the tissue of the aortic arch roof, encircling the plurality of the ostia of the aortic side branch vessels inside the aortic arch, covering its entrance. The 3-dimensional structure of the filter membrane is designed so that when deployed it should acquire the anatomical form of the aortic arch, thus avoiding hindering the passage of catheters and devices through the aortic arch.

The filter membrane manufactured according to the disclosure herein is part of an embolic protection device, wherein the filter membrane is arranged to separate a first fluid volume of the aortic side branch vessels from a second fluid volume in the aortic arch when the protection unit is positioned in the aortic arch.

The mesh of the filter membrane may be made of an elastic and atraumatic material such as a polymer.

In some examples, the obtained 3-dimensional structure may be dome-shaped, such as illustrated in figs 1A and 1B. The 3-dimensional structure, such as a dome-shape, may have other shapes than the shape illustrated herein, it may for example, be a section of a sphere, a section of a ellipsoid, a section of a paraboloid, a section of an obloid etc.

A filter membrane 1000, 1100 having a 3-dimensional structure, such as a dome-shape, may improve the space underneath the embolic protection device. A filter membrane 1000, 1100 having a 3-dimenstional structure, such as a dome-shape, may improve the filtering due to a larger filter area.

A disadvantage of most filter membranes in the prior art is that they may use too much fabric to allow the device to be collapsed by stretching or crimping for loading into a delivery device and to provide a large filtering area. When using too much fabric, the embolic protection device will be unnecessarily large when collapsed requiring either a larger size of a delivery device or the device will be harder to release from the delivery device. another issue is the use of too little fabric for the filter membrane which may constrain the embolic protection when expanded after delivery and preventing an optimal sealing against the walls of the aortic arch. Some prior art devices used mechanical members, such as struts or ribs, to archive a 3-dimensional structure, such as a dome shape. This makes the embolic protection devices complicated larger than needed when collapsed for delivery.

The filter membranes in Figs. 1A and 1B have two portions before being mounted on a support member. A first portion 10 and a second portion 11. Between the first portion 10 and the second portion 11 is a periphery 12 of the first portion.

The manufacturing method disclosed herein, allows a 3-dimenstional structure, such as a dome-shape, to be made from a 2-dimensional sheet of woven mesh, for example a polymer, such as Polyetereterketon (PEEK). 3-dimensional structure is obtained without any cuts or welding to provide a 3-dimensional structure without any stitches or welded segments. Hence the structure may have no overlaps, creases or other distortions that may affect the performance of the embolic protection device.

The first portion 10 of the filter membrane 1000, 1100 is obtained by the manufacturing method without significant changing the porosity factor, such as the mesh size, or change the structure of the material, such as elongation or stretching of the strands or having the strands at least partly melted. Hence the filtering performance and the flexibility, such as crimpability of the embolic protection device will not be affected.

The obtained filter membrane 1000, 1100 has a 3-dimensional structure, such as a dome-shape, may be mounted on a support member added adjacent the periphery 12, either before the second portion 12 is removed or after.

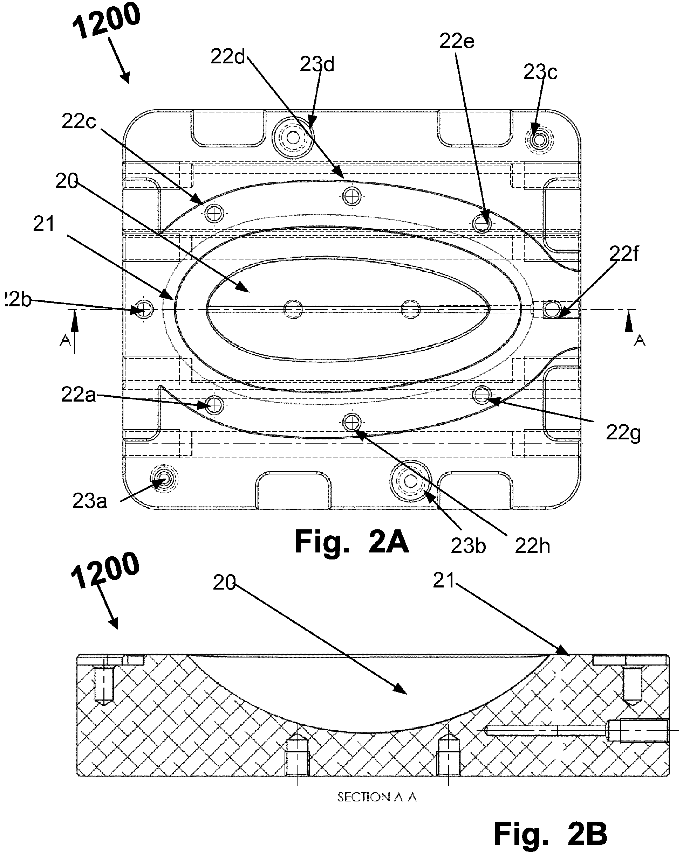

The mold used for obtaining the 3-dimensional structure includes two parts. Figs. 2A and 2B are illustrating a first part 1200 of the mold. Fig 2A is a top view of the first part 1200 while Fig. 2B is a section along A-A.

The first part 1200 of the mold has a cavity 20 shaped as the 3-dimensional structure to be obtained. The first part of the mold 1200 has a surface 21 that circumscribe the cavity 20.

In some examples, the surface 21 may have pegs or holes 22a-h for attaching the sheet of fabric to for aligning the strands of the fabric to the cavity during the heat treatment. The pegs or holes 22a-h may additionally and/or alternatively be used for aligning the two parts of the mold. Additional and/or alternatively, in some examples, pegs and/or holes 23a-d may be used for aligning the two parts of the mold.

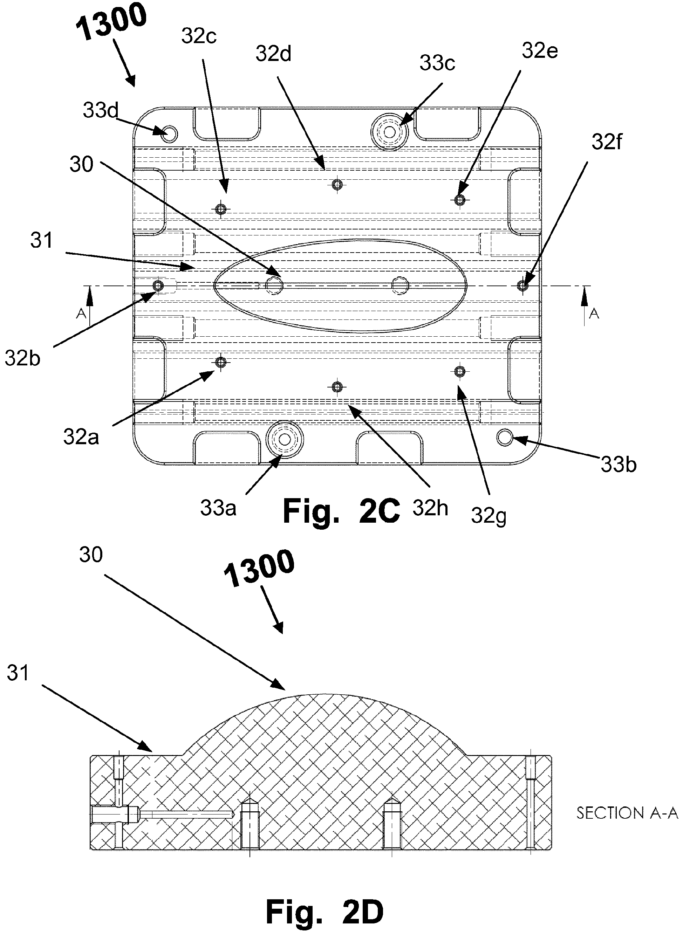

Figs. 2C and 2D are illustrating a second part 1300 of the mold. Fig 2C is a top view of the second part 1300 while Fig. 2D is a section along A-A.

The Second part 1300 of the mold has a protruding portion 30 shaped as the 3-dimensional structure to be obtained. The second part of the mold 1300 has a surface 31 that circumscribe the protruding portion 30.

In some examples, the surface 31 may have pegs or holes 32a-h for attaching the sheet of fabric to for aligning the strands of the fabric to the cavity during the heat treatment. The pegs or holes 32a-h may additionally and/or alternatively be used for aligning the two parts of the mold. Additional and/or alternatively, in some examples, pegs and/or holes 33a-d may be used for aligning the two parts of the mold.

In some examples of the mold has the surface 21 that circumscribe the cavity 20 of the first part 1200 pegs 22a-h for alignment of the sheet of fabric, and the surface 31 of the second part 1300 that circumscribe the protruding portion 30 has corresponding holes, 32a-h. In some other examples has the first part 1200 of the mold holes 22a-h and the second part 1300 of the mold has corresponding pegs 31a-h. In some additional examples have the first part 1200 both holes and pegs 22a-h and the second part 1300 of the mold may has corresponding pegs and holes 32a-h.

The same applies to the second set of holes and pegs 23a-d of the first part 1200 which have corresponding pegs and holes at the second part 1300 of the mold.

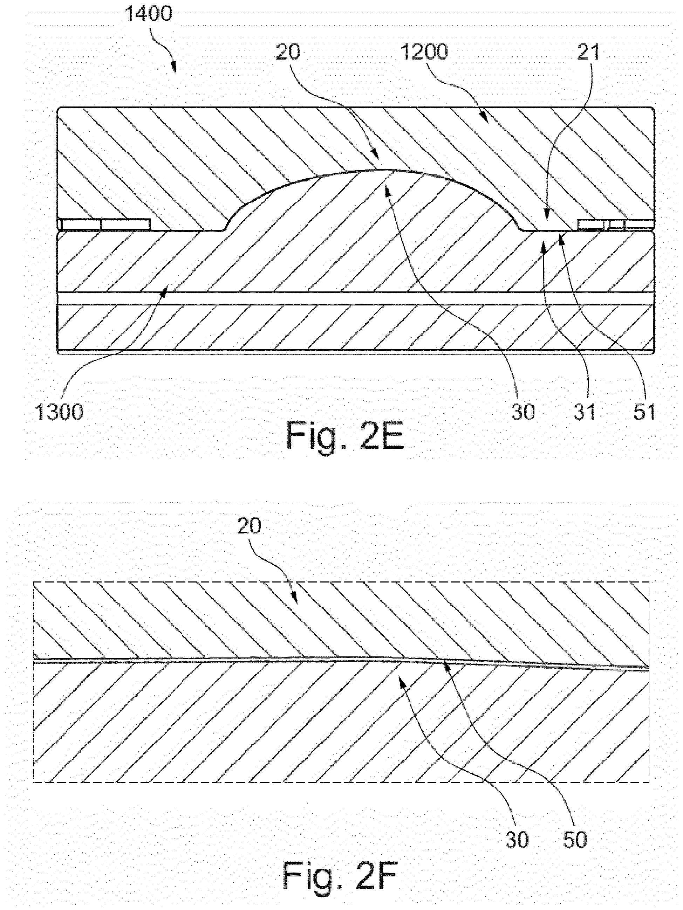

Figs. 2E and 2F are illustrating a mold 1400 wherein the first part 1200 and the second part 1300 are put together. As illustrated, there is no space 51 between the surface 21 that circumscribe the cavity 20 and the surface 31 that circumscribe the protruding portion 30. Further, it is illustrated that there is a distance 50 between the protruding portion 30 and the cavity 20. This distance is obtained by making the cavity slightly larger and/or the protruding portion slightly smaller than the 3-dimensional structure to be obtained. The distance may be between a few microns and a couple of millimeters. In some examples, the distance may be larger than the thickness of a fabric to be arranged there between.

Fig. 2F is illustrating a magnification of the gap 50 between the protruding part 30 and the cavity 20.

The distance between the first cavity 20 and the protruding portion 30 allows there to be no pressure on the part of the fabric positioned there between during the heat treatment. As there is no pressure applied on the fabric positioned between the cavity and the protruding portion, there may be no change in the properties or characteristics material, such as by elongating or stretching of the strands of the fabric. Further, the strands of the fabric will not be separated and the porosity factor, such as the mesh size, will be maintained.

In some examples, there is a pressure applied to the first portion on the fabric positioned between the cavity and the protruding portion, the pressure applies is smaller than the pressure applies on the portion of the sheet of fabric positioned between the circumscribing surfaces 21, 31

The portion of the sheet of fabric positioned between the circumscribing surfaces 21, 31 may have changed properties, such as the material at this portion of the sheet being at least partly melted or at least partly annihilated, as this portion of the fabric is under pressure applied by the weight of the part of the mold arranged on top of the other.

In some additional examples, additional pressure is applied on the portion of the fabric arranged between the surfaces 21, 31, for example by having members forcing the two parts 1200, 1300 against each other, such as screws, pneumatic piston, weight, or any other method know to the person skilled in the art for exerting a pressure on a mold.

The sheet is then heat treated by applying a temperature during a period of time. After the temperature has been applied, the sheet of fabric may be allowed to cool for over a period of time before being the mold is opened and the filter is removed.

The temperature and time depend on the material. For PEEK, the temperature may be between 150 and 250 degrees Celsius, such as 190 to 210 degrees Celsius, such as 200 degrees Celsius. The time may range from seconds up to a couple of minutes, such as up to 1 minute, such as between 10 and 30s, such as between 15 and 25s, such as 20 to 25s.

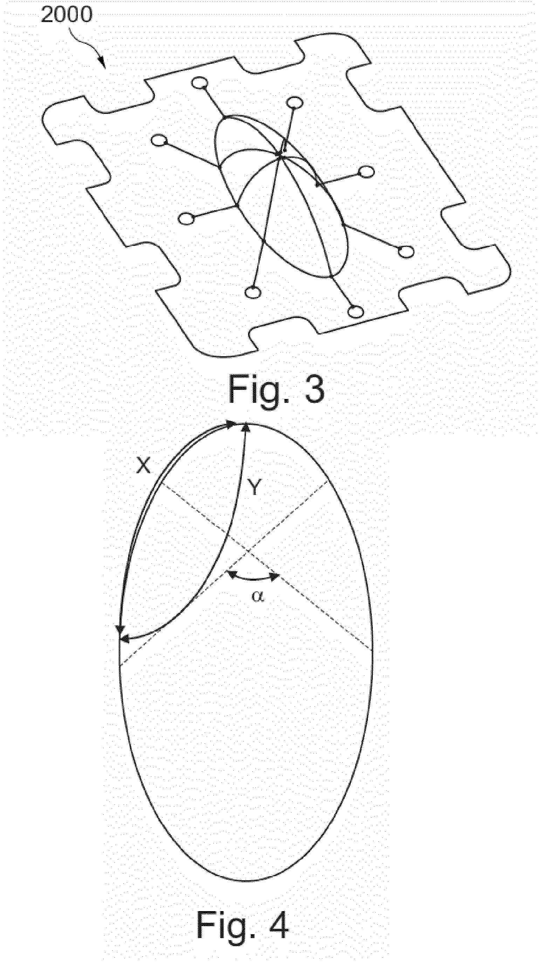

Fig. 3 is illustrating an example of determining the amount of fabric needed in the mold before heat-treatment. Fig. 3 is illustrating a piece of flat fabric 2000, such as a mesh. The amount of fabric is determined by calculating distances between discrete points creating enough extra material in the center when heat setting the structure the extra material left will help to result in a 3-dimensional structure, such as a dome, with no elongated or separated strands. Hence the porosity factor of the filter member will be kept and the 3-dimensional structure will be uniform.

Fig. 4 is illustrating exemplary characteristic of a heat-treated 3D-strucuture. In some examples, to achieve optimal flexibility when it comes to stretching and crimping the embolic protection device and at the same time keep the amount of material at the minimum but still avoiding the risk of the filter membrane constraining the device, the distance x between two points along the periphery of the 3-dimensional structure should be about the same as the distance Y between the same two points over the 3-dimensional structure. This relationship makes it possible to have a 3-dimenstional structure that collapse by the support member without constraining the device it.

Further, in some example, a filter member or mesh may be configured from woven strands wherein the yarn orientation is at angles α that are not at right angles to the periphery or the 3-dimensional structure or a support member of an embolic protection device. In some examples, the mesh may be aligned when arraigning the sheet of fabric in the mold so that when the filter membrane has been mounted on the support member, the weave (warp and weft) of the mesh or weave may be, for example, at angles α of 45° from a base or lateral portion of the support frame. In some examples, the weave (warp and weft) of mesh may be at for example 30-60°, such as 35-55°, angles α from a base or lateral portion of the support frame. When set at a non-right angle to the support frame, the mesh may stretch, expand or contract with greater flexibility than when such weave is at right angles to the support frame. Collapsibility or crimpability of the embolic protection device is advantageously improved in this manner.

Hence it is care has to be taken during the heat setting to avoid stretching and movement of the strands in the fabric which may affect the angles α between the warp and the weft.

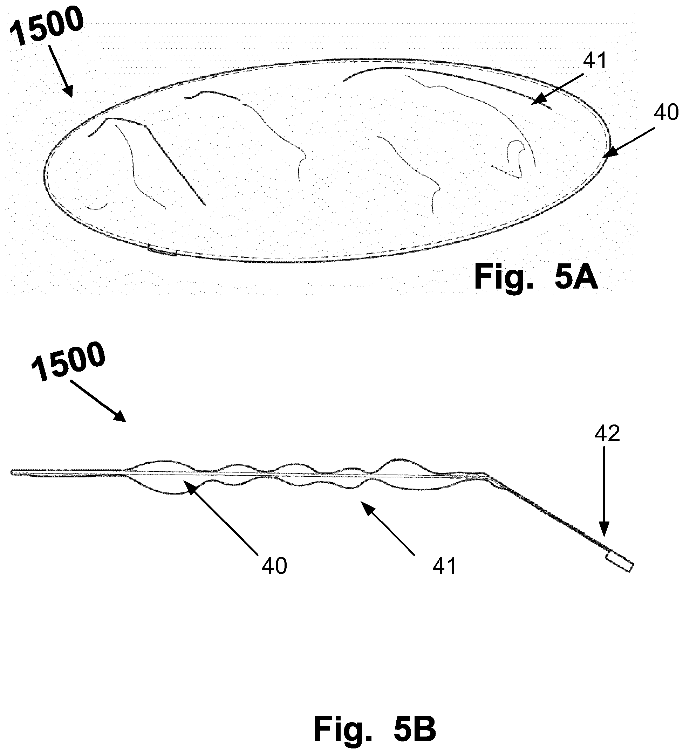

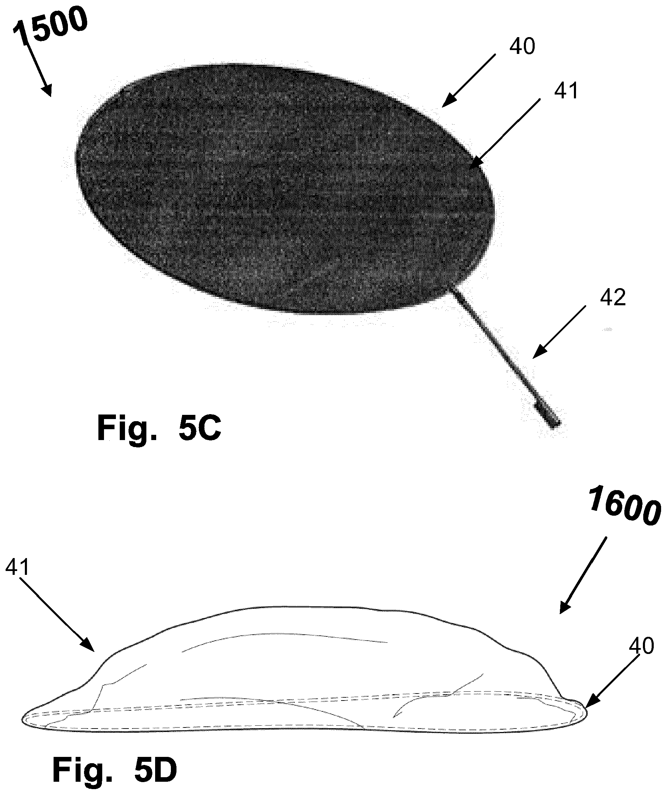

Figs. 5A to 5D are illustrating a schematic example of an embolic protection device 1500, 1600 in accordance with the invention, having a filter membrane with a 3D-structure when unconstrained 1500 and constrained 1600.

Figs. 5A to 5C are illustrating an embolic protection device 1500 when unconstrained outside of an aortic arch. The embolic protection device is collapsible, such as crimpable, to be arranged in a transvascular delivery unit. The protection device 1500 includes a support member 40 and a filter member 41 attached to the support member 40. The support member 40 may be, in some examples, a complete hoop completely surrounding a periphery of the filter member 41. In some examples, the filter member 41 may extend (partly or entirely) outside the periphery defined by support fame 40, and thereby create a collar or rim. The collar or rim may improve apposition with the vessel wall rough texture. In some examples, the collar or rim may be made from a different material than the filter member 41.

The protection device 1500 may further include a connection point 42 which may be at the support member 40. The connection point 42 is used for connecting the embolic protection device 1500 to a transvascular delivery unit. Preferably the connection point 42 is arranged at a proximal portion of the embolic protection device 1500. In some examples, a connection point 42 may be arranged on an elongated member, such as a stem, at distance from the filter membrane 41 and the support frame 40.

When the embolic protection device 1500 is unconstrained, the support member 40 will retain its original shape and the filter member 41 will become almost completely flat, ass illustrated in figs 5A to 5C.

In the illustrated example, the support member 40 is a ring but may in some examples have a more elongated shape, such as oblong or elliptic.

Fig. 5D is illustrating an embolic protection device 1600 where the support member 40 is slightly constrained whereby the filter member 41 strives to obtain its heat set 3-dimensional structure, such as a dome-shape.

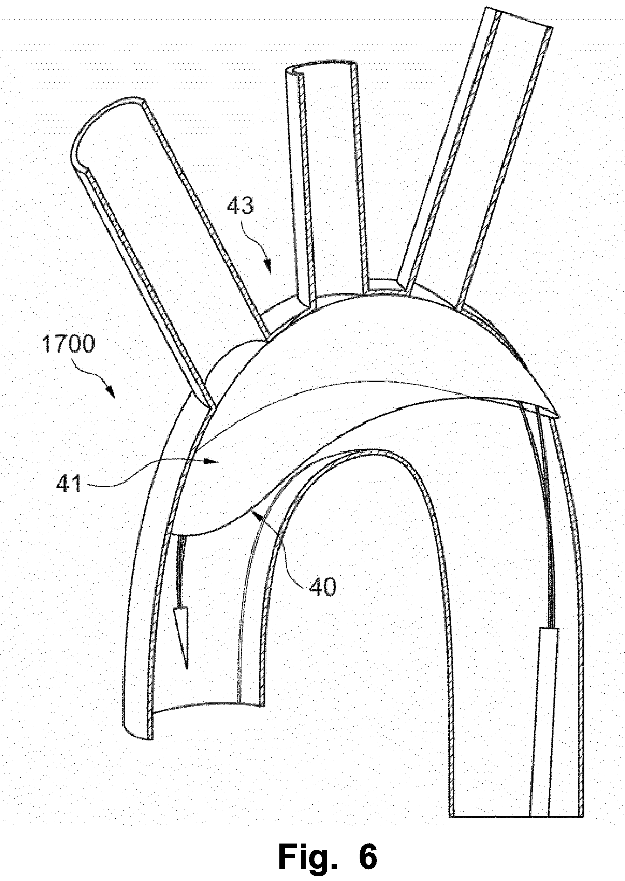

These properties of the embolic protection device 1700 makes it possible to have a support member 40 being larger than most of the prior art devices and that may self-align in the aortic arch 42 at a lower location than most of the embolic protection devices in the prior art, as illustrated in Fig. 6. Even thou the frame member having a larger area, self-expanding 3-dimenstional structure will line the inner wall of the aortic arch above the support member. Hence the embolic protection device may span over the whole apex from the ascending aorta to the descending aorta of the aortic arch preventing emboli from entering any of the side branches. The larger filtering area will improve the f improve the filtering and provide a better sealing against the walls of the aortic arch.

For positioning a protection device 1700 in an aorta, the device 1700 of the disclosure may be attached to and delivered by a transvascular delivery unit, for example as illustrated in Fig. 1B. The transvascular delivery unit may be, for example, a catheter or sheath, and the protection device 1700 may be attached to the transvascular delivery unit according to methods known in the art, or by a connector mechanism. In some examples, the transvascular delivery unit may comprise a connector mechanism 20, such as a wire, rod or tube, for example, a tether, a delivery wire, or a push wire etc. The connector mechanism may be connected to the connection point. In some examples, the connector mechanism may be permanently connected to the embolic protection device 1700. Thereby the embolic protection device 1700 may be delivered and withdrawn using the same connector mechanism. Further, the connector mechanism may be used to hold the embolic protection device 1700 in place during a medical procedure. In some examples, the connector mechanism may be detachably connected to the embolic protection device 1700.

The distal end and/or the proximal end of the support frame may be made from a spring section. Each spring section may be a pre-loaded spring that function as an engine and is configured for quickly expand or open-up a collapsed or crimped embolic protection device 1700 from a collapsed state to an expanded state and for providing a radial force between the support member 40 and a wall of the aortic arch 43, when the support member 40 is in an expanded state. The spring sections are engines being pre-shaped open springs. The spring sections may have a radius wider than the embolic protection device 1700. Different radius of the opening may provide different forces.

The spring sections may provide improved apposition with aortic arch walls which may improve fixation of the device 1700 and the sealing between the device and the wall of the aorta, which may reduce paraframe leakage. The force from the spring sections may also avoid distortion of the support member 1700 when a radial force is applied. The force from the spring sections also tends to position the embolic protection device 1700 at about mid-vessel diameter. Hence provides an embolic protection device with improved self-positioning and alignment properties.

The force provided by the spring sections may also reduce windsailing, in most cases to none.

The spring sections are preferably heat treated to form the spring sections and to provide spring properties. The spring sections are in some examples, formed as open springs and are wider than the protection device before the device is assembled.

By arranging a spring section proximally, there will be an improved coverage of the landing zone. The landing zone is the area every guidewire will hit aortic arch. An improved coverage and sealing of the landing zone may help to prevent the passage of devices over (along) the protection device 1700 (through the aortic arch), for example by leading a guide wire below the protection device 1700.

Each spring section has a bend shape, such as a shallow U-shape, or is curved. In examples where the support member 40 only has one spring section at either the distal or the proximal end, the rest of the support member 40 has a deeper u-shaped form. This deeper U-shaped form does not have the same springy properties as the spring section. In examples where the support member has a spring section at both the distal and the proximal ends, the support member may have straight central sections formed between spring sections. When using straight central sections, these are substantially straight before the device is assembled. After the device is assembled, the straight central sections may bulge or obtain a curvature due to forces in the support frame from the spring sections.

In some examples, the straight central sections may function as spring engines in a longitudinal direction of the embolic protection device.

Additionally, and/or alternatively, in some examples, the spring sections are heat treated to form the spring sections, while the rest of the support member is not heat treated. This will give the support ember 40 a flexibility that may further improve apposition of the embolic protection device 1700 with the aortic arch walls as it complies better with the rough texture of the vessel wall.

Further, by heat treating all sections there may be forces at the transitions between the segments, such as at joints between segments, applicable to the wall of the aortic arch. Also, if the wire is made from a single wire being heat treated, there will be fewer connectors for joining the different sections, which may also improve the forces from the transitions between the segments to the wall of the aortic arch.

An advantage of only heat treating the spring sections and not the other sections, is that the forces from the spring sections will be comparatively stronger.

To further improve the force, some segments may be made thicker than others, for example, at the distal end of the support frame 10, the distal spring section may be thicker than the rest of the support frame, and weaker proximally. This may also make it easier to crimp the support member 40, e.g. into a catheter lumen for delivery, or for improved exiting such lumen when deploying the embolic protection device.

Alternatively, in some examples, both the distal and the proximal spring sections are made thicker than the rest of the support frame. This will improve the spring forces at both the proximal and the distal end. The thicker spring sections may open up the support frame while the thinner sections are more compliant with the vessel wall.

Alternatively, in some examples, both the spring sections and the central sections are made thicker than the joints or transition segment(s) between the thicker sections that may be made thinner. This will provide strong forces on all sides while avoiding the issues of making the whole support frame rigid. Making the whole support frame rigid may force the spring sections to close and not efficiently cover tortuous anatomies with the embolic protection device.

By utilizing different thicknesses or cross sections of different sections, a support frame may be obtained having a configuration with different forces at different segments. Additionally, and or alternatively, the at least distal or proximal spring section may include a spring element. The spring element may in some examples be a loop or helix, a small spring or any other type of spring arranged at about the centre of each of the distal or proximal spring section. The spring element is used for increasing the force applied by the support member 40 on the walls of the aortic arch 43.

As previously described, the spring sections 12, 13 are used for applying a force by the support frame 10 on the wall of aortic arch which may improve the sealing effect between the collapsible embolic protection device and the wall of the aortic arch, as well as provide an improved self-stabilizing effect. Additionally, the use of spring sections 12, 13 may improve the positioning and self-alignment of the device in the aortic arch.

Additionally, and/or alternatively, in some examples, the connector mechanism may be attached to the support frame 10 allowing the protection device to pivot axially but not radially at the joint between the support frame and the connector mechanism, for example by attaching the connector element via the proximal loop.

The spring element, especially the proximal spring element 14, may in some examples function as a crimp element to improve the collapsibility of the embolic protection device by elongating the device longitudinally. Thereby allows to embolic protection device 1000 to be crimped into a sheath with small diameter.

Spring elements may in some examples, for example when the spring elements are loops, be formed to either protruding outwards (relative the periphery/footprint defined by the support frame) or formed to be protruding inwards (relative the periphery/footprint defined by the support frame). Arranging or forming one or more of the spring elements to protrude inwards improves attachment of the filter member 41 to the support member 40. Also, having one or more of the spring elements arranged to protrude inwards improves the contact between the support member 40 and the walls of the aortic arch 43 as there is nothing protruding or extending further than the support member 40 (smooth apposition to the aortic wall vessel tissue, further improvable by a collar mentioned herein).

The support member 40 may be made from a wire, such as a spring wire, or being laser cut from a tube, ribbon, sheet, or flat wire, etc. The support member 40 may be of a single wire. In some examples, the support member 40 is made from a twisted single wire. Alternatively, in some examples the support member 40 may be made of at least two wires being twisted, braided or knitted.

The support member 40 may be in some examples made from joint free ring. In other examples, the support member 40 made be formed from a ring having at least one joint. A joint may be for example a fixation like a soldering, welding, or a clamp.

The support member 40 may be shaped into an elongated shape, substantially elliptical, oblong, oval, or cone slot shaped. Alternatively, other shapes may be used, such as circular or rectangular. Because the aortic anatomy can vary between individuals, examples of the intra-aortic device of the disclosure may be shaped to adapt to a variety of aortic anatomies.

The size of the collapsible device may be pre-sized and pre-formed to accommodate various patient groups (e.g., children and adults) or a particular aortic anatomy. The support member 40 may be, in some examples, substantially planar. In some examples, the support member 40 may have a width greater than the diameter of the aortic arch into which it is configured to be positioned in use, such as about 50% greater than the diameter of the aortic arch, such as 50 % greater than the cross-sectional chord of an aorta of a subject, in which the collapsible embolic protection device 1700 may be placed. Additionally, in some examples, a support member 40 may be longer than the aortic arch opening, such as about 20% longer than the arch opening, such as 20 % longer than an approximate distance between an upper wall of an ascending aorta of a subject, distal to an opening of an innominate artery, and an upper wall of a descending aorta of a subject, proximal to an opening of a left subclavian artery.

By making the support member 40 wider than the diameter of the arch, such as about 50% wider, and longer than the aortic arch opening, such as about 20% longer, as defined above, the self-positioning of the device positioning about mid vessel diameter may be improved and thus improve the apposition with aortic arch walls. This will make it easier to deploy the embolic protection device and improve the sealing against the walls. It may also improve the coverage of all three side vessels, innominate (brachiocephalic) artery, left common carotid artery, or left subclavian artery) which are supplying blood to the brain.

The support member 40 may be fabricated in whole or in part from, e.g., nitinol or metal, superelastic or shape memory alloy material, readily malleable material, or polymer, e.g., nylon. The metal may include, e.g., tantalum or platinum.

The filter member 41 prevents particles (e.g., emboli) typically having a dimension between about 50 µm and about 5 mm (e.g., 50 pm, 100 pm, 200 pm, 300 pm, 400 pm, 500 pm, 750 pm, 1 mm, 2 mm, 3 mm, 4 mm, or 5 mm) in an aorta from passing into blood vessels (e.g., innominate (brachiocephalic) artery, left common carotid artery, or left subclavian artery) supplying blood to the brain. Accordingly, one or more lateral dimensions of the pores of the filter can be between about 50 µm and about 5 mm (e.g., 50 pm, 100 pm, 200 pm, 300 pm, 400 pm, 500 pm, 750 pm, 1 mm, 2 mm, 3 mm, 4 mm, or 5 mm). The filter may be, e.g., a mesh made from a plurality of fibers made of polymer, nylon, nitinol, or metal, or a combination thereof. The mesh may be made from woven fibers. Fibers may be from about 20 to 50 µm in thickness. According to a configuration not covered by the invention, the filter may be a perforated film. When a perforated film is present, the pores formed in the perforated film may include pores of varied or unvaried shape (e.g., rectilinear or rhomboid pores), have a varied or constant density across the film, and/or have a constant or varied size. The size of the pores of the filter allows passage of blood cells (e.g., red blood cells (erythrocytes), white blood cells (leukocytes), and/or platelets (thrombocytes)) and plasma, while being impermeable to particles (e.g., emboli) larger than the pore dimensions. Emboli filtered by the mesh of the filter of the present disclosure are typically particles larger in one or more dimensions than an aperture of the mesh of the filter.

Various catheters or sheath may be used as part of the present disclosure. Any catheter or sheath known in the art to be configured for guiding medical instruments through vasculature may be used (e.g., stent installation catheter, ablation catheter, or those used for transcatheter aortic valve implantation (TAVI) or percutaneous aortic valve replacement (PAVR) procedures, e.g., as described in U.S. Patent No. 5,026,366). Additionally, and/or alternatively, the device may include a pigtail catheter, which may be of size 6F or smaller (e.g., 1F, 2F, 3F, 4F, 5F, or 6F) and include a radiopaque material to facilitate tracking the progress of various elements of the device. Other catheters that can be used as part of the disclosure include any catheter used in procedures associated with a risk of embolism, which would benefit by including an intravascular filter as part of the procedure.

A device of the disclosure may incorporate radiopaque elements. Such radiopaque elements can be affixed to or incorporated into the device. For example, portions of the frame, filter, or catheter may be constructed of OFT wire. Such wire can contain, e.g., a core of tantalum and/or platinum and an outer material of, e.g., nitinol.

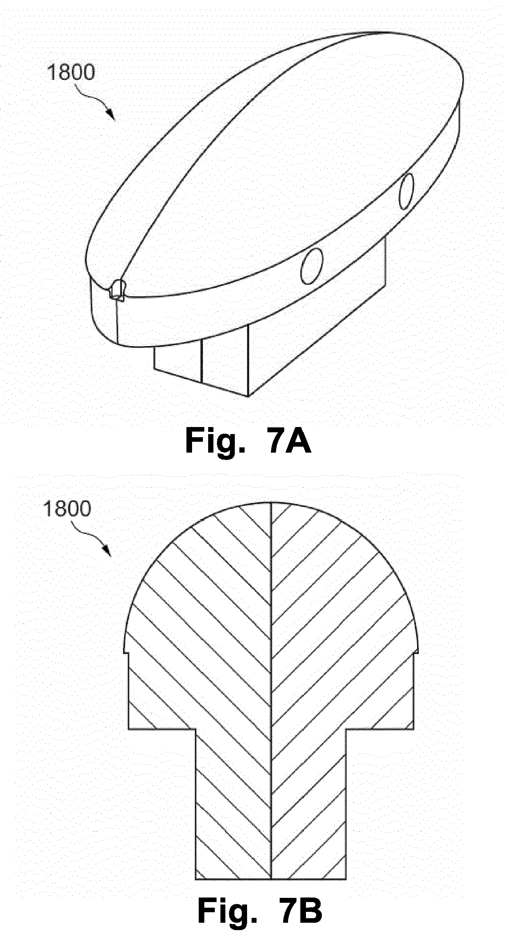

Figs. 7A and 7B are illustrating a jig 1800 with a design to hold the 3-dimentional structures shaped filter membrane, in this case a dome shaped mesh. The supporting element e.g. frame, is then constrained to a shape of the conferencing the base of the 3-dimensional structure and supported while applying an adhesive e.g. UV adhesive or any other kind of bonding material between the support frame and the filter membrane, while maintaining the 3-dimensional shape of the filter membrane.

Any excessive material of the second portion of the filter membrane may be removed after the filter membrane has been mounted on the support frame. In this way, the support member will be arranged at the right position in relation to the 3-dimensional structure to make it possible for the filter membrane to return to its pre-set shape when the support frame is constrained by the walls of the aortic arch. Alternatively, in some examples, the support member may be in its extended shape and the filter membrane is stretched over the support frame before an adhesive e.g. UV adhesive or any other kind of bonding material is applied between the filter membrane and the support frame.

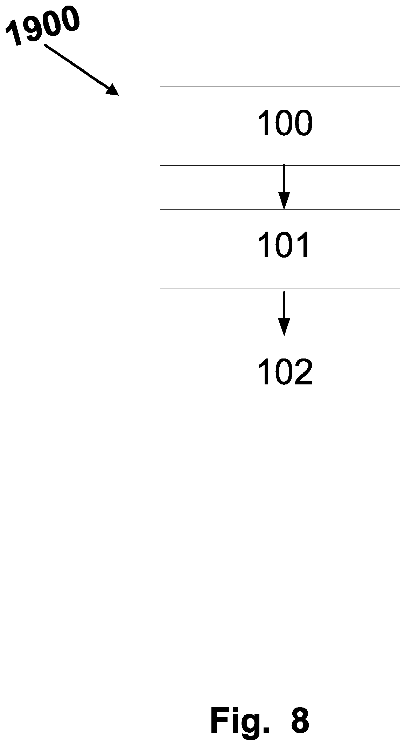

Fig. 8 is illustrating a chart 1900 over a method of manufacturing an emboli filter having a three-dimensional (3D) structure from a sheet of fabric made from at least of strand.

Arranging 100 a sheet of fabric in a mold comprising two parts, a first part of a mold having a cavity in the shape of the three-dimensional structure surrounded by a first surface, and a second part having a protrusion in the shape of the three-dimensional structure surrounded by a second surface. The sheets may be arranged on the first part, or alternatively on the second part.

Closing 101 the mold. When the mold is closed there is a space between a surface of the cavity and a surface of the protruding portion. A first portion of the sheet of fabric will be arranged in the space between the surface of the cavity and the surface of the protruding portion and a second portion will be arranged between the surfaces surrounding the cavity and the protruding portion. The second portion will be exposed to a pressure asserted by the first and second part of the mold.

Heat treating 102 the fabric by elevating the temperature in the mold until a set-temperature is reached. Hold the temperature at the set-temperature for a period of time. The heat will be switched of and the fabric may be allowed to cool in the mold before being removed.

After the fabric has been removed from the device it may be mounted of a support member. The fabric may be mounted on the support fame while the support frame is held flat in a jig in a slightly compressed state. The filter membrane may be adhered to the support frame by using glue- The filter membrane may also be attached to the support frame using heat welding, ultrasonic welding, or stitching, etc. The skilled person would readily appreciate that there are other options known in the art for attaching a filter membrane to a support frame.

While several examples of the present disclosure have been described and illustrated herein, those of ordinary skill in the art will readily envision a variety of other means and/or structures for performing the functions and/or obtaining the results and/or one or more of the advantages described herein, and each of such variations and/or modifications is deemed to be within the scope of the present disclosure. More generally, those skilled in the art will readily appreciate that all parameters, dimensions, materials, and configurations described herein are meant to be exemplary and that the actual parameters, dimensions, materials, and/or configurations will depend upon the specific application or applications for which the teachings of the present disclosure is/are used. Also, different method steps than those described above, performing the method by hardware, may be provided within the scope of the disclosure. The different features and steps of the disclosure may be combined in other combinations than those described. The scope of the disclosure is only limited by the appended patent claims.

## Claims

1. An embolic protection device for deployment in an aortic arch of a patient for protection of side branch vessels of the aortic arch from embolic material, the device comprising:
a filter membrane (1000, 1100, 41) comprising a sheet of fabric made from at least one strand, and
wherein said filter membrane having a 3D-structure (10) obtained by a manufacturing process comprising the following steps:
providing a mold made from two parts, a first part having a cavity in the shape of the 3D-structure surrounded by a first surface, a second part having a protrusion in the shape of the 3D-structure surrounded by a second surface, and when the mold is closed there is a space between a surface of the cavity and a surface of the protruding portion;
arranging the sheet of fabric between said two parts of said mold so that a first portion of said sheet is arranged between said surface of said cavity and said surface of said protruding part, and a second portion of said sheet circumscribe said first portion is arranged between said first and second surface;
molding said sheet of fabric by heat treatment to set said sheet of fabric;
the embolic protection device further comprises a support frame (40) arranged along a periphery of said 3D-structure (10); wherein said support frame (40) stretches said filter membrane (1000, 1100, 41) to become substantially flat when unconstrained; and
wherein said filter membrane (1000, 1100, 41) obtains said 3D-structure (10) when said support frame (40) is constrained.

2. The device of claim 1, wherein said sheet of fabric is made of polyetheretherketon (PEEK).

3. The device of any of claims 1 to 2, wherein said sheet of fabric is molded to have a distance between two points along a periphery of said 3D-structure to be the same as a distance between the same two points over the 3D-structure (10).

4. The device of any of claims 1 to 3, wherein said support frame (40) is a hoop completely surrounding said periphery of said filter member (1000, 1100, 41).

5. The device of any of claims 1 to 4, wherein a connection point (42) for connecting the embolic protection device to a transvascular delivery unit is arranged at said support frame (40) at a proximal portion thereof.

6. The device of any of claims 1 to 4, wherein a connection point (42) for connecting the embolic protection device to a transvascular delivery unit at a distance from the filter membrane and the support frame is arranged on an elongated member attached to said support frame (40) at a proximal portion of said embolic protection device.

7. The device of any of claims 1 to 6, wherein said support frame (40) is a wire shaped into a ring.

8. The device of any of claims 1 to 7, wherein said embolic protection device is sized to span over an apex of said aortic arch from an ascending aorta to a descending aorta.

9. The device of any of claims 1 to 8, wherein a distal end and/or a proximal end of said support frame (40) is made of a spring section.

10. The device of claim 9, wherein the spring section is a pre-loaded spring that function as an engine and is configured for quickly expand or open-up the collapsed or crimped embolic protection device from a collapsed state to an expanded state and for, during use, provide a radial force between said support frame (40) and a wall of said aortic arch when said support frame is in said expanded state.

11. The device of any of claims 1 to 10, wherein said 3D-structure (10) has no stiches or welded segments.

## Patentansprüche

1. Eine Embolieschutzprothese zur Implementierung in einem Aortenbogen eines Patienten zum Schutz von Seitengefäßen des Aortenbogens vor embolischen Material, wobei die Vorrichtung Folgendes umfasst:
eine Filtermembran (1000, 1100, 41) mit einem Gewebetuch, das aus mindestens einem Faden gefertigt wurde und
wobei besagte Filtermembran eine 3D-Struktur (10) aufweist, die über ein Herstellungsverfahren mit folgenden Schritten erzielt wird:
Bereitstellung einer Form aus zwei Teilen, wobei der erste Teil eine der 3D-Struktur entsprechende und von einer ersten Oberfläche umgebene Hohlform aufweist, ein zweiter Teil einen der 3D-Struktur entsprechenden und von einer zweiten Oberfläche umgebenen Vorsprung und wobei bei geschlossener Form ein Spalt zwischen der Oberfläche des Hohlraums und der Oberfläche des vorstehenden Bereichs vorhanden ist,
die Anordnung des Gewebetuchs zwischen den beiden Teilen der besagten Form, so dass ein erster Abschnitt besagten Tuchs zwischen besagter Oberfläche besagten Hohlraums und besagter Oberfläche besagten vorstehenden Teils angeordnet ist und ein zweiter Abschnitt besagten Gewebes den besagten ersten Abschnitt begrenzt und zwischen erster und zweiter Oberfläche angeordnet ist,
das Formen besagten Stoffgewebes durch eine Wärmebehandlung des besagten Stoffgewebes,
die Embolieschutzprothese weist weiterhin einen Stützrahmen (40) auf, der entlang des Rands besagter 3D-Struktur (10) angeordnet ist, wobei besagter Stützrahmen (40) besagte Filtermembran (1000, 1100, 41) streckt, so dass sie in unbelastetem Zustand im Wesentlichen flach ist, und
wobei besagte Filtermembran (1000, 1100, 41) besagte 3D-Struktur (10) ausbildet, wenn besagter Stützrahmen (40) belastet wird.

2. Die Vorrichtung gemäß Anspruch 1, wobei besagtes Gewebetuch aus Polyetheretherketon (PEEK) besteht.

3. Die Vorrichtung gemäß einem der Ansprüche 1 bis 2, wobei besagtes Gewebetuch geformt wird, so dass es eine Distanz zwischen zwei Punkten am Rand besagter 3D-Struktur aufweist, die der Distanz zwischen den beiden gleichen Punkten auf der 3D-Struktur entspricht.

4. Die Vorrichtung gemäß einem der Ansprüche 1 bis 3, wobei besagter Stützrahmen (40) einem Band entspricht, das besagten Rand besagten Filterelements (1000, 1100, 41) komplett umgibt.

5. Die Vorrichtung gemäß einem der Ansprüche 1 bis 4, wobei ein Verbindungspunkt (42) zum Anschluss der Embolieschutzprothese an eine transvaskuläre Einführungseinheit an einem proximalen Abschnitt besagtem Stützrahmen (40) angebracht ist.

6. Die Vorrichtung gemäß einem der Ansprüche 1 bis 4, wobei ein Verbindungspunkt (42) zum Anschluss der Embolieschutzprothese an eine transvaskuläre Einführungseinheit in einem Abstand zur Filtermembran und der Stützrahmen an einem länglichen Element angeordnet sind, das an besagtem Stützrahmen (40) in einem proximalen Abschnitt besagter Embolieschutzprothese befestigt ist.

7. Die Vorrichtung gemäß einem der Ansprüche 1 bis 6, wobei der besagte Stützrahmen (40) einem ringförmigen Draht entspricht.

8. Die Vorrichtung gemäß einem der Ansprüche 1 bis 7, wobei besagte Embolieschutzprothese so bemessen ist, dass sie sich über die Spitze besagten Aortenbogens hinweg von einer aufsteigenden Aorta bis zu einer absteigenden Aorta erstreckt.

9. Die Vorrichtung gemäß einem der Ansprüche 1 bis 8, wobei ein distales Ende und/oder ein proximales Ende besagten Stützrahmens (40) einen Federabschnitt umfasst.

10. Die Vorrichtung gemäß Anspruch 9, wobei es sich bei dem Federabschnitt um eine vorgespannte Feder handelt, die als Antrieb dient und zur schnellen Ausdehnung oder Öffnung der zusammengefallenen oder gefalteten Embolieschutzprothese aus dem zusammengefallenen Zustand in einen ausgefahrenen Zustand ausgelegt ist, sowie zur Bereitstellung einer radialen Kraft während des Gebrauchs zwischen besagtem Stützrahmen (40) und einer Wand besagten Aortenbogens, wenn besagter Stützrahmen sich im ausgefahrenen Zustand befindet.

11. Die Vorrichtung gemäß einem der Ansprüche 1 bis 10, wobei der besagte 3D-Struktur (10) keine Stiche oder geschweißten Segmente aufweist.

## Revendications

1. Dispositif de protection embolique destiné à un déploiement dans un arc aortique d'un patient pour la protection de vaisseaux de ramification latérale de l'arc aortique contre un matériau embolique, le dispositif comprenant :
une membrane filtrante (1000, 1100, 41) comprenant une feuille de tissu constitué à partir d'au moins un fil, et où ladite membrane filtrante présentant une structure en 3D (10) obtenue par un procédé de fabrication comprenant les étapes consistant à :
fournir un moule constitué de deux parties, une première partie présentant une cavité ayant la forme de la structure en 3D entourée d'une première surface, une seconde partie présentant une protubérance ayant la forme de la structure en 3D entourée d'une seconde surface, et lorsque le moule est refermé, il existe un espace entre une surface de la cavité et une surface de la partie protubérante ;
agencer la feuille de tissu entre lesdites deux parties dudit moule de sorte qu'une première portion de ladite feuille est agencée entre ladite surface de ladite cavité et ladite surface de ladite partie protubérante, et une seconde portion de ladite feuille entourant ladite première portion est agencée entre lesdites première et seconde surfaces ;
mouler ladite feuille de tissu par traitement thermique pour façonner ladite feuille de tissu ;
le dispositif de protection embolique comprend en outre un cadre de support (40) agencé le long d'une périphérie de ladite structure en 3D (10) ; où ledit cadre de support (40) étire ladite membrane filtrante (1000, 1100, 41) pour la rendre sensiblement plane lorsqu'il n'est pas soumis à contrainte ; et où ladite membrane filtrante (1000, 1100, 41) obtient ladite structure en 3D (10) lorsque ledit cadre de support (40) est soumis à contrainte.

2. Dispositif selon la revendication 1, dans lequel ladite feuille de tissu est constituée de polyétheréthercétone (PEEK).

3. Dispositif selon l'une quelconque des revendications 1 et 2, dans lequel ladite feuille de tissu est moulée pour présenter une distance entre deux points le long d'une périphérie de ladite structure en 3D identique à une distance entre les mêmes deux points en travers de la structure en 3D (10).

4. Dispositif selon l'une quelconque des revendications 1 à 3, dans lequel ledit cadre de support (40) est un cerceau entourant entièrement ladite périphérie de ladite membrane filtrante (1000, 1100, 41).

5. Dispositif selon l'une quelconque des revendications 1 à 4, dans lequel un point de liaison (42) destiné à relier le dispositif de protection embolique à une unité de pose transvasculaire est agencé au niveau dudit cadre de support (40) au niveau d'une portion proximale de celui-ci.

6. Dispositif selon l'une quelconque des revendications 1 à 4, dans lequel un point de liaison (42) destiné à relier le dispositif de protection embolique à une unité de pose transvasculaire à une certaine distance de la membrane filtrante et du cadre de support est agencé sur un élément allongé attaché audit cadre de support (40) au niveau d'une portion proximale dudit dispositif de protection embolique.

7. Dispositif selon l'une quelconque des revendications 1 à 6, dans lequel ledit cadre de support (40) est un câble façonné en un anneau.

8. Dispositif selon l'une quelconque des revendications 1 à 7, où ledit dispositif de protection embolique est dimensionné pour s'étendre sur un apex dudit arc aortique depuis une aorte montante jusqu'à une aorte descendante.

9. Dispositif selon l'une quelconque des revendications 1 à 8, dans lequel une extrémité distale et/ou une extrémité proximale dudit cadre de support (40) est constituée d'une section de ressort.

10. Dispositif selon la revendication 9, dans lequel ladite section de ressort est une ressort préchargé qui fonctionne comme un moteur et est configuré pour déployer ou ouvrir rapidement le dispositif de protection embolique écrasé ou replié d'un état écrasé vers un état déployé et pour, pendant l'utilisation, fournir une force radiale entre ledit cadre de support (40) et une paroi dudit arc aortique lorsque ledit cadre de support se trouve dans ledit état déployé.

11. Dispositif selon l'une quelconque des revendications 1 à 10, dans lequel ladite structure en 3D (10) ne présente pas de sutures ni de segments soudés.
